# FASCICULE DE BREVET EUROPEEN

(11) **EP 0 682 939 B1**
(45) Date de publication et mention de la délivrance du brevet: **14.11.2001**
(21) Numéro de dépôt: 95400924.7
(22) Date de dépôt: 25.04.1995
(51) Int. Cl.: A61K 7/48, A61K 7/06, A61K 7/04

(54) **Utilisation d'une suspension colloidale à base de charges minérales comme composition cosmétique permettant de former un film sur les cheveux, la peau et/ou les ongles**
Verwendung einer mineralischen kolloidalen Suspension als filmbildendes Kosmetikum auf Haaren, Haut und Nageln
Use of a mineral colloidal suspension as filmforming cosmetic on hair, skin and nails

(30) Priorité: 28.04.1994 FR 9405181
(43) Date de publication de la demande: 22.11.1995
(73) Titulaire: L'OREAL, 75008 Paris (FR)
(72) Inventeur: Caisey, Laurence, F-94400 Vitry-sur-Seine (FR); Sturla, Jean-Michel, F-92210 Saint-Cloud (FR)
(74) Mandataire: Casalonga, Axel

(56) Documents cités:
- EP-A- 0 244 859
- EP-A- 0 261 560
- GB-A- 1 342 091
- US-A- 4 255 416
- DATABASE WPI Week 77, Derwent Publications Ltd., London, GB; AN 7756501 & JP-A-52 076 439 (HINOKI SHINYAKU)

## Description

La présente invention a pour objet l'utilisation de suspensions colloïdales à base de charges minérales dans le traitement cosmétique de la peau, des cheveux ou des ongles en vue de former, après évaporation du milieu solvant, un film minéral sur ces matières.

Des suspensions colloïdales à base de charges minérales sont connues en elles-mêmes et ont déjà été utilisées dans l'état de la technique, en particulier pour conférer, notamment à des substrats tels que des matières plastiques ou vitreux, des propriétés antireflets (WO 93/04386 ou FR-A-2 693 558) ou encore pour réaliser des miroirs diélectriques interférentiels (WO 93/08490).

On a déjà utilisé des sols de dioxyde de titane comme matières premières dans la réalisation de compositions absorbantes destinées à protéger la peau contre les rayonnements UV.

La demande de brevet EP 0 261 560 décrit un procédé de préparation d'un sol d'oxyde de titane présentant un pH neutre pouvant être dispersé dans une base cosmétique en vue d'une utilisation comme anti-solaire.

La demanderesse a découvert, ce qui fait l'objet de l'invention, que l'utilisation des suspensions colloïdales à base de charges minérales permettait d'obtenir, après évaporation du solvant, un film minéral. Ce film permet en particulier de modifier les propriétés réfléchissantes de la peau, des cheveux ou des ongles. C'est ainsi que la peau traitée avec une suspension colloïdale appropriée peut être rendue plus mate, ce qui a pour conséquence d'atténuer de façon visuelle les rides. Les cheveux traités avec une telle composition peuvent présenter une brillance améliorée. Ces suspensions confèrent par ailleurs à la peau, aux cheveux et/ou aux ongles de bonnes propriétés cosmétiques.

Les suspensions colloïdales utilisables conformément à l'invention, sont en particulier préparées par le procédé sol-gel connu en lui-même.

L'invention a donc pour objet l'utilisation de suspensions colloïdales à base de charges minérales comme composition cosmétique, en vue de former après évaporation un film minéral sur la peau, les cheveux ou les ongles.

Un autre objet de l'invention est constitué par les compositions mises en oeuvre dans le cadre de cette utilisation.

L'invention a également pour objet l'utilisation de ces compositions en vue de modifier les propriétés réfléchissantes des cheveux, de la peau ou des ongles.

D'autres objets de l'invention apparaîtront à la lecture de la description et des exemples qui suivent.

L'invention est plus particulièrement relative à l'utilisation d'une suspension colloïdale à base de charges minérales pouvant être préparée par le procédé sol-gel comme composition cosmétique pour le traitement de la peau, des cheveux et/ou des ongles, ladite suspension contient dans un milieu cosmétique constitué par de l'eau, un mélange eau-alcool ou un alcool, des sels ou oxydes de métaux sous forme de particules, ayant un indice de réfraction d'au moins 1,9 ou compris entre 1,3 et 1,6 inclus, pour former après évaporation du milieu cosmétique un film minéral modifiant les propriétés réfléchissantes de la peau, des cheveux et/ou des ongles.

La suspension utilisée conformément à l'invention contient, dans un milieu constitué par de l'eau, un mélange eau-alcool ou un alcool, au moins un sel ou un oxyde de métal, sous forme de particules présentant un indice de réfraction compris entre 1,3 et 1,6 ou supérieur ou égal à environ 1,9, de préférence compris entre 1,9 et 3.

Grâce à la composition conforme à l'invention, il est possible de modifier les propriétés réfléchissantes de la peau, des cheveux et/ou des ongles par une augmentation de la brillance ou en conférant plus de matité aux cheveux, à la peau ou aux ongles.

La demanderesse a constaté que l'on pouvait augmenter la brillance des cheveux en utilisant une suspension colloïdale contenant un sel ou un oxyde de métal ayant un indice de réfraction supérieur à 1,9, de préférence supérieur à 2,1, en particulier entre 1,9 et 2,9.

La demanderesse a constaté également que cet aspect brillant persiste même après coiffage et le film déposé sur les cheveux laisse aux cheveux un toucher naturel, les cheveux traités ne collent pas et présentent par ailleurs des propriétés cosmétiques parfaitement acceptables.

Les propriétés de matité sont plus particulièrement obtenues et intéressantes pour le traitement de la peau. C'est ainsi que la demanderesse a constaté que l'utilisation de suspensions colloïdales contenant des sels ou oxydes de métaux ayant un indice de réfraction compris entre 1,3 et 1,6 et de préférence entre 1,35 et 1,50, conférait à la peau une matité sans blanchiment, la peau est moins brillante, présente moins de reflets, ce qui a pour avantage d'atténuer les rides. Ces effets restaient même après rinçage.

Les oxydes de métaux plus particulièrement utilisables, conformément à l'invention, sont choisis parmi TiO₂, Al₂O₃, Ta₂O₅, Nb₂O₅, ThO₂, ZrO₂, HfO₂, ainsi que leurs hydrates tels que Ta₂O₅.2,6H₂O, Nb₂O₅.2,3H₂O, Al₂O₃.H₂O. Ces oxydes de métaux permettent en particulier d'apporter la brillance à la peau, aux cheveux et aux ongles.

D'autres oxydes ou sels de métaux utilisables sont constitués par SiO₂, MgF₂, CaF₂ qui présentent plus particulièrement l'avantage de conférer à la peau un caractère mat. On constate en effet une atténuation visuelle de la réflexion, un effet de masquage des rides et des défauts de surface sans blanchiment de la peau.

Le film déposé sur les cheveux, la peau et/ou les ongles est poreux, confère à ces supports un indice de réfraction compris entre 1,15 et 1,35, ce qui a pour effet de donner plus de matité ou entre 1,65 et 2,9, ce qui a pour effet d'améliorer la brillance des matières traitées. Ces indices peuvent être modifiés en agissant sur la porosité du film déposé.

Ce film est constitué essentiellement d'oxydes ou de sels de métaux tels que définis ci-dessus.

On utilise plus particulièrement ces charges minérales sous forme de particules ayant une dimension comprise entre 1 nm et 300 nm, et plus préférentiellement entre 5 et 100 nm.

Ces particules peuvent avoir une forme sphérique ou parallélépipédique, ou autre.

Les charges minérales plus particulièrement préférées, conformément à l'invention, sont le dioxyde de titane (TiO₂) qui confère un effet de brillance aux cheveux et SiO₂ qui donne un effet matifiant, en particulier à la peau.

Les suspensions colloïdales ont généralement une viscosité comprise entre 1 et 5 mPa.s.

Les compositions conformes à l'invention sont connues en elles-mêmes et peuvent être préparées par des procédés bien connus dans l'état de la technique. Ces compositions sont plus particulièrement préparées à partir de sels ou d'alcoxydes des métaux correspondants solubilisés dans un solvant, tels qu'un alcool. On procède ensuite à une réaction d'hydrolyse pour former un précipité amorphe. On disperse ensuite avec un acide ou une base selon le pH souhaité, ce qui conduit à la peptisation du précipité et à la cristallisation. On forme de cette façon un oxyde cristallin dans un solvant.

De telles suspensions colloïdales peuvent être préparées selon des procédés connus en eux-mêmes, tels que décrits dans J. Colloïd Interface Sci., 26, p. 62-69, 1968 pour SiO₂, Appli. Opt. 26, 4688, 1987 pour TiO₂, Inorg. Chem., 3, 146, 1964 pour ZrO₂ et HfO₂, de U.S.-A-3.256.204, 1966 pour ThO₂, de Am. Cer. Soc. Bull. 54, 289, 1975 pour AtOOH, MRS, Better Ceramics Through Chemistry, 1991 pour Ta₂O₅ et Nb₂O₅, Appl. Opt., 27, 3356, 1988 pour CaF₂ et MgF₂.

Ces suspensions sont préparées en utilisant des précurseurs ioniques choisis le plus souvent parmi les chlorures, les oxychlorures, les perchlorates, les nitrates, les oxynitrates ou encore les acétates ou des précurseurs moléculaires de préférence choisis parmi les alcoxydes, de formule molaire M(OR)ₙ (M représentant un métal, OR un radical alcoxy de 1 à 6 atomes de carbone et n représentant la valence du métal. Dans les méthodes décrites précédemment, le précurseur est hydrolysé ou fluoré puis polymérisé jusqu'à l'obtention d'un produit fini, insoluble dans le solvant choisi, nucléé et appelé suspension colloïdale. Dans le cas des alcoxydes, l'hydrolyse doit être rigoureusement contrôlée étant donné le caractère très hydrophile de ces dérivés organométalliques.

Un autre procédé de préparation de tels produits est décrit dans J. LIVAGE et autres, "SOL-GEL SYNTHESIS OF METAL OXYDE CLUSTERS AND COLLOIDS" (MAT. RES. SOC. SYNT. PROC., Volume 272, pages 3 à 14).

Les suspensions colloïdales utilisables conformément à l'invention, ont généralement une concentration en charges minérales en suspension comprise entre 0,001% et 25%, et de préférence entre 0,05% et 10% en poids par rapport au poids total de la suspension colloïdale.

Le milieu utilisable peut être constitué par de l'eau ou un mélange eau-alcool ou par un alcool. Les alcools sont choisis plus particulièrement parmi les alcools inférieurs ayant entre 1 et 4 atomes de carbone, tels que l'éthanol, l'isopropanol ou des polyols tels que le propylèneglycol, la glycérine et le sorbitol.

Le pH de ces compositions est compris généralement entre 5 et 10, de préférence entre 6 et 9 et peut être ajusté par des agents alcalinisants ou acidifiants cosmétiquement acceptables, connus en eux-mêmes.

Les compositions conformes à l'invention peuvent également contenir différents additifs, de préférence non-ioniques tels que plus particulièrement des agents tensio-actifs non-ioniques comme les alkylphénoxypolyéthoxyéthanols tels que l'octylphénoxypolyéthoxy-éthanol, le nombre de groupements éthoxy étant compris entre 2 et 10.

Les compositions peuvent également contenir des silicones, des polymères non-ioniques tels que l'alcool polyvinylique, la polyvinylpyrrolidone, le polyvinylbutyral, des glycérols.

On peut également additionner à ces compositions des colorants ayant pour but de colorer la composition ou les cheveux ou la peau, des agents antiradicaux libres, des agents hydratants, des filtres solaires pour protéger la peau contre les effets des rayonnements UV, ainsi que tout autre additif qui n'a pas d'effet déstabilisant de la suspension colloïdale ou sur le film que forme cette suspension colloïdale au niveau des cheveux, de la peau et/ou des ongles.

Ces compositions peuvent contenir, comme colorants, des colorants directs ou des pigments, tels que les pigments dérivés de la mélanine, dans des proportions comprises entre 0,1 et 10% par rapport aux charges minérales utilisées conformément à l'invention. On peut plus particulièrement utiliser les pigments mélaniques dérivés en particulier de l'oxydation du 5,6-dihydroxyindole ou de ses dérivés ainsi que des pigments mélaniques d'origine naturelle ou encore de l'oxyde de fer coloré.

Les compositions utilisables conformément à l'invention peuvent être appliquées sous forme d'aérosol, de gel ou de lotion. L'application de ces produits peut être suivie ou non d'un rinçage.

Le produit utilisé conformément à l'invention est pulvérisé sur les cheveux. On constate, après séchage, la formation d'un film qui, lorsque la charge minérale est constituée par un agent ayant un indice de réfraction supérieur à 1,9, donne une brillance plus importante tout en conférant aux cheveux des propriétés cosmétiques telles que de douceur au toucher, démêlage intéressantes.

Pour traiter la peau, on peut pulvériser à partir d'un flacon pompe la suspension colloïdale. Après séchage et rinçage éventuel, on constate lorsque la charge minérale mise en oeuvre a un indice de réfraction inférieur à 1,6 , moins de réflexion et une atténuation des rides.

Les exemples suivants sont destinés à illustrer l'invention sans pour autant présenter un caractère limitatif.

### EXEMPLE 1

On applique par pulvérisation sur les cheveux une suspension aqueuse, colloïdale, contenant 4,5% en poids de dioxyde de titane (TiO₂), la viscosité de cette suspension est de 1,18 mPa.s et son pH est de 10. Les particules de TiO₂ ont une granulométrie moyenne d'environ 22 nm.

Après pulvérisation, on laisse sécher. Un film fin est déposé. On met en forme les cheveux et on constate que les cheveux ont un aspect brillant qui persiste après coiffage. Les cheveux ont par ailleurs un toucher naturel qui ne colle pas et possèdent de bonnes propriétés cosmétiques.

### EXEMPLE 2

On pulvérise sur la peau une suspension colloïdale comportant 3,5% de SiO₂ de granulométrie 26 nm dans de l'éthanol. Ce sol a un pH de 7,5. Après séchage, on constate que la peau a un aspect beaucoup plus mat qui a pour conséquence d'atténuer les rides. La peau garde sa couleur et ne blanchit pas.

### EXEMPLE 3

On prépare la composition suivante :
- TiO₂ (granulométrie moyenne 29 nm) 3% en poids,
- Eau qs 100 g
- pH = 9

Cette composition est appliquée par pulvérisation sur les ongles. On constate une augmentation de la brillance des ongles après séchage. Cet effet est maintenu même après rinçage avec de l'eau.

### EXEMPLE 4

On prépare la composition suivante :
- TiO₂ 3% en poids
- Polyvinylpyrrolidone 0,3%
- Eau qsp 100 g

On applique cette composition sur les cheveux. Les cheveux, une fois séchés, sont brillants et cet effet de brillance se maintient après coiffage et mise en forme.

## Revendications

1. Utilisation d'une suspension colloïdale à base de charges minérales pouvant être préparée par le procédé sol-gel comme composition cosmétique pour le traitement de la peau, des cheveux et/ou des ongles, **caractérisée en ce que** ladite suspension contient dans un milieu cosmétique constitué par de l'eau, un mélange eau-alcool ou un alcool, des sels ou oxydes de métaux sous forme de particules, ayant un indice de réfraction d'au moins 1,9 ou compris entre 1,3 et 1,6 inclus, pour former après évaporation du milieu cosmétique un film minéral modifiant les propriétés réfléchissantes de la peau, des cheveux et/ou des ongles.

2. Utilisation selon la revendication 1, **caractérisée en ce que** l'on utilise des oxydes de métaux choisis parmi TiO₂, Al₂O₃, Ta₂O₅, Nb₂O₅, ThO₂, ZrO₂, HfO₂, ainsi que leurs hydrates pour conférer à la peau et/ou aux cheveux et/ou aux ongles des propriétés de brillance.

3. Utilisation selon la revendication 1, **caractérisée en ce que** l'on utilise comme sel ou oxydes de métaux SiO₂, MgF₂, ou CaF₂ en vue de conférer à la peau et/ou aux cheveux un aspect mat.

4. Utilisation selon l'une quelconque des revendications 1 ou 2, **caractérisée en ce que** l'on utilise comme oxyde de métal TiO₂ en vue d'augmenter la brillance des cheveux.

5. Utilisation selon l'une quelconque des revendications 1 ou 2, **caractérisée en ce que** l'on utilise comme oxyde de métal SiO₂ en vue d'atténuer de façon visuelle les rides sur la peau.

6. Procédé de traitement cosmétique de la peau, des cheveux et/ou des ongles, **caractérisé en ce que** l'on applique sur la peau, les cheveux et/ou les ongles une suspension colloïdale à base de charges minérales pouvant être préparée par le procédé sol-gel contenant dans un milieu cosmétique constitué par de l'eau, un mélange eau-alcool ou un alcool, des sels ou oxydes de métaux sous forme de particules, ayant un indice de réfraction d'au moins 1,9 ou compris entre 1,3 et 1,6 inclus, et **en ce que** ladite suspension permette après évaporation du milieu l'obtention d'un film minéral modifiant les propriétés réfléchissantes de la peau, des cheveux et/ou des ongles.

7. Procédé selon la revendication 6, **caractérisé en ce que** la suspension colloïdale est telle que définies dans l'une quelconque des revendications 2 à 5.

8. Procédé selon l'une quelconque des revendications 6 ou 7 **caractérisé en ce que** l'on forme sur la peau un film ayant un indice de réfraction compris entre 1,15 et 1,35 et entre 1,65 et 2,9.

9. Procédé selon l'une quelconque des revendications 6 à 8 **caractérisé en ce que** les suspensions colloïdales contiennent entre 0,001% et 25% d'oxydes ou de sels de métaux et de préférence entre 0,05% et 10% en poids par rapport au poids total de la suspension.

10. Procédé selon l'une quelconque des revendications 6 à 9 **caractérisé en ce que** les particules d'oxydes ou de sels de métaux ont une granulométrie moyenne comprise entre 1 et 300 nm et de préférence entre 5 et 100 nm.

11. Procédé selon l'une quelconque des revendications 6 à 10 **caractérisé en ce que** l'on confère aux cheveux de la brillance par la mise en oeuvre d'une suspension colloïdale contenant un oxyde de métal choisi parmi TiO₂, Al₂O₃, Ta₂O₅, Nb₂O₅, ThO₂, ZrO₂, HfO₂ ou ses hydrates.

12. Procédé selon l'une quelconque des revendications 6 à 10 destiné à donner un aspect mat et atténuer visuellement les rides de la peau, **caractérisé en ce que** l'on utilise comme oxydes ou sels de métaux SiO₂, MgF₂, ou CaF₂.

13. Procédé selon l'une quelconque des revendications 6 à 12, **caractérisé en ce que** l'on pulvérise la suspension colloïdale sur les cheveux, la peau et/ou les ongles et que l'on laisse sécher.

14. Procédé selon la revendication 13, **caractérisé en ce que** l'on procède à un rinçage de la peau et/ou des cheveux après séchage de la suspension colloïdale appliquée.

## Patentansprüche

1. Verwendung einer kolloidalen Suspension auf der Basis von anorganischen Füllstoffen, die durch das Sol-Gel-Verfahren hergestellt werden kann, als kosmetische Zusammensetzung zur Behandlung der Haut, der Haare und/oder der Nägel,
**dadurch gekennzeichnet, daß**
die Suspension in einem kosmetischen Medium, das aus Wasser, einem Wasser-Alkohol-Gemisch oder einem Alkohol besteht, Metallsalze oder Metalloxide in Form von Partikeln enthält, die einen Brechungsindex von mindestens 1,9 oder im Bereich von 1,3 bis 1,6 aufweisen, um nach dem Verdampfen des kosmetischen Mediums einen anorganischen Film zu bilden, der die Reflexionseigenschaften der Haut, der Haare und/oder der Nägel verändert.

2. Verwendung nach Anspruch 1, **dadurch gekennzeichnet, daß** Metalloxide verwendet werden, die unter TiO₂, Al₂O₃, Ta₂O₅, Nb₂O₅, ThO₂, ZrO₂ und HfO₂ sowie ihren Hydraten ausgewählt sind, um der Haut und/oder den Haaren und/oder den Nägeln Glanzeigenschaften zu verleihen.

3. Verwendung nach Anspruch 1, **dadurch gekennzeichnet, daß** als Metallsalze oder Metalloxide SiO₂, MgF₂ oder CaF₂ verwendet werden, um der Haut und/oder den Haaren ein mattes Aussehen zu verleihen.

4. Verwendung nach einem der Ansprüche 1 oder 2, **dadurch gekennzeichnet, daß** als Metalloxid TiO₂ verwendet wird, um den Glanz des Haares zu erhöhen.

5. Verwendung nach einem der Ansprüche 1 oder 2, **dadurch gekennzeichnet, daß** als Metalloxid SiO₂ verwendet werden, um sichtbar die Falten der Haut zu mildern.

6. Verfahren zur kosmetischen Behandlung der Haut, der Haare und/oder der Nägel, **dadurch gekennzeichnet, daß** auf die Haut, die Haare und/oder die Nägel eine kolloidale Suspension auf der Basis von anorganischen Füllstoffen aufgebracht wird, die nach dem Sol-Gel-Verfahren hergestellt werden kann und in einem kosmetischen Medium, das aus Wasser, einem Wasser-Alkohol-Gemisch oder einem Alkohol besteht, Metallsalze oder Metalloxide in Form von Partikeln enthält, die einen Brechungsindex von mindestens 1,9 oder im Bereich von 1,3 bis 1,6 aufweisen, und dadurch, daß durch die Suspension nach dem Verdampfen des Mediums ein anorganischer Film erhalten wird, der die Reflexionseigenschaften der Haut, der Haare und/oder der Nägel verändert.

7. Verfahren nach Anspruch 6, **dadurch gekennzeichnet, daß** es sich bei der kolloidalen Suspension um eine Suspension nach einem der Ansprüche 2 bis 5 handelt.

8. Verfahren nach einem der Ansprüche 6 oder 7, **dadurch gekennzeichnet, daß** auf der Haut ein Film mit einem Brechungsindex von 1,15 bis 1,35 und 1,65 bis 2,9 ausgebildet wird.

9. Verfahren nach einem der Ansprüche 6 bis 8, **dadurch gekennzeichnet, daß** die kolloidalen Suspensionen 0,001 bis 25 Gew.-% Metalloxide oder Metallsalze und vorzugsweise 0,05 bis 10 Gew.-%, bezogen auf das Gesamtgewicht der Suspension, enthalten.

10. Verfahren nach einem der Ansprüche 6 bis 9, **dadurch gekennzeichnet, daß** die Partikel von Metalloxiden oder Metallsalzen eine mittlere Korngröße von 1 bis 300 nm und vorzugsweise 5 bis 100 nm aufweisen.

11. Verfahren nach einem der Ansprüche 6 bis 10, **dadurch gekennzeichnet, daß** den Haaren Glanz verliehen wird, indem eine kolloidale Suspension verwendet wird, die ein Metalloxid enthält, das unter TiO₂, Al₂O₃, Ta₂O₅, Nb₂O₅, ThO₂, ZrO₂, HfO₂ oder deren Hydraten ausgewählt ist.

12. Verfahren nach einem der Ansprüche 6 bis 10, das dazu vorgesehen ist, zu mattieren und die Falten der Haut sichtbar abzuschwächen, **dadurch gekennzeichnet, daß** als Metalloxid oder Metallsalz SiO₂, MgF₂ oder CaF₂ verwendet wird.

13. Verfahren nach einem der Ansprüche 6 bis 12, **dadurch gekennzeichnet, daß** die kolloidale Suspension auf den Haaren, der Haut und/oder den Nägeln zerstäubt und trocknen gelassen wird.

14. Verfahren nach Anspruch 13, **dadurch gekennzeichnet, daß** die Haut und/oder die Haare nach dem Trocknen der aufgebrachten kolloidalen Suspension gespült wird.

## Claims

1. Use of a colloidal suspension based on inorganic fillers which can be prepared by the sol-gel process as a cosmetic composition for treating the skin, hair and/or nails, the said suspension contains [sic], in a cosmetic medium consisting of water, a water/alcohol mixture or an alcohol, metal salts or oxides in the form of particles, having a refractive index of at least 1.9 or between 1.3 and 1.6 inclusive, in order to form, after evaporation of the cosmetic medium, an inorganic film which modifies the reflective properties of the skin, hair and/or nails.

2. Use according to Claim 1, **characterized in that** metal oxides chosen from TiO₂, Al₂O₃, Ta₂O₅, Nb₂O₅, ThO₂, ZrO₂ or HfO₂, and their hydrates, are used to confer properties of lustre/sheen/gloss on the skin and/or hair and/or nails.

3. Use according to Claim 1, **characterized in that** SiO₂, MgF₂ or CaF₂ is used as metal salts or oxides for the purpose of conferring a matt appearance on the skin and/or hair.

4. Use according to either one of Claims 1 and 2, **characterized in that** TiO₂ is used as metal oxide for the purpose of increasing the sheen of the hair.

5. Use according to either one of Claims 1 and 2, **characterized in that** SiO₂ is used as metal oxide for the purpose of visually disguising wrinkles on the skin.

6. Process for cosmetic treatment of the skin, hair and/or nails, **characterized in that** a colloidal suspension based on inorganic fillers which can be prepared by the sol-gel process, containing, in a cosmetic medium consisting of water, a water/alcohol mixture or an alcohol, metal salts or oxides in the form of particles, having a refractive index of at least 1.9 or between 1.3 and 1.6 inclusive, is applied to the skin, hair and/or nails and **in that** the said suspension makes it possible, after evaporation of the medium, to obtain an inorganic film which modifies the reflective properties of the skin, hair and/or nails.

7. Process according to Claim 6, **characterized in that** the colloidal suspension is such as are defined [sic] in any one of Claims 2 to 5.

8. Process according to either one of Claims 6 and 7, **characterized in that** a film having a refractive index of between 1.15 and 1.35 and between 1.65 and 2.9 is formed on the skin.

9. Process according to any one of Claims 6 to 8, **characterized in that** the colloidal suspensions contain between 0.001 % and 25 % of metal oxides or salts and preferably between 0..05 % and 10 % by weight with respect to the total weight of the suspension.

10. Process according to any one of Claims 6 to 9, **characterized in that** the particles of metal oxides or salts have a mean particle size of between 1 and 300 nm and preferably between 5 and 100 nm.

11. Process according to any one of Claims 6 to 10, **characterized in that** sheen is conferred on the hair by the use of a colloidal suspension containing a metal oxide chosen from TiO₂, Al₂O₃, Ta₂O₅, Nb₂O₅, ThO₂, ZrO₂ or HfO₂ or its hydrates.

12. Process according to any one of Claims 6 to 10, intended to give a matt appearance and visually to disguise wrinkles of the skin, **characterized in that** SiO₂, MgF₂ or CaF₂ is used as metal oxides or salts.

13. Process according to any one of Claims 6 to 12, **characterized in that** the colloidal suspension is sprayed onto the hair, skin and/or nails and **in that** it is left to dry.

14. Process according to Claim 13, **characterized in that**, after drying of the applied colloidal suspension, the skin and/or hair is/are rinsed.
